# EUROPÄISCHE PATENTSCHRIFT

(11) **EP 2 101 900 B1**
(45) Veröffentlichungstag und Bekanntmachung des Hinweises auf die Patenterteilung: **23.04.2014**
(21) Anmeldenummer: 07857723.6
(22) Anmeldetag: 17.12.2007
(51) Int. Cl.: B01F 5/04, B01J 8/08, B01J 8/12, C07C 5/333, C07B 35/04, C07C 5/48

(54) **REAKTOR ZUR DURCHFÜHRUNG EINER KONTINUIERLICHEN OXIDEHYDRIERUNG SOWIE VERFAHREN**
REACTOR FOR CARRYING OUT A CONTINUOUS OXIDE HYDROGENATION, AND METHOD
RÉACTEUR POUR RÉALISER UNE DÉSHYDROGÉNATION OXYDANTE EN CONTINU ET PROCÉDÉ ASSOCIÉ

(30) Priorität: 19.12.2006 DE 102006060509; 19.12.2006 US 870715 P
(43) Veröffentlichungstag der Anmeldung: 23.09.2009
(73) Patentinhaber: BASF SE, 67056 Ludwigshafen (DE)
(72) Erfinder: OLBERT, Gerhard, 69221 Dossenheim (DE); CORR, Franz, 67067 Ludwigshafen (DE); CRONE, Sven, 67117 Ludwigshafen (DE)
(86) Internationale Anmeldenummer: PCT/EP2007/064093
(87) Internationale Veröffentlichungsnummer: WO 2008/074787

(56) Entgegenhaltungen:
- DE-A1- 2 222 562
- DE-A1- 3 240 987
- DE-A1-102004 032 129
- US-A- 2 350 644
- US-A- 2 417 393
- US-A- 3 217 247
- US-A- 4 102 776
- US-A- 5 462 719

## Beschreibung

Die Erfindung betrifft einen Reaktor zur Durchführung einer kontinuierlichen Oxidehydrierung eines Einsatzgasstromes von gesättigten Kohlenwasserstoffen sowie ein Verfahren zur Durchführung einer kontinuierlichen Oxidehydrierung im Reaktor.

Oxidehydrierungen werden in der chemischen Verfahrenstechnik im großtechnischen Umfang zum Aufwerten von paraffinhaltigen Kohlenwasserstoffströmen zu den entsprechenden Olefinen durchgeführt. Da die Reaktion endotherm ist, muss Wärme von außen zugeführt werden. Großtechnische Oxidehydrierungen sind beispielsweise die Oxidehydrierung von Propan oder die Oxidehydrierung von Butan.

Nach dem als UOP-Oleflex-Prozess bekannten Verfahren zur Dehydrierung von Propan zu Propen wird ein Propan enthaltender Einsatzgasstrom auf eine erhöhte Temperatur, von häufig 700 bis 750°C, vorgeheizt, und in Wanderbett-Dehydrierreaktoren an einem Katalysator, der Platin auf Aluminiumoxid enthält, dehydriert, wobei ein überwiegend Propan, Propen und Wasserstoff enthaltender Produktgasstrom erhalten wird. Nach dem UOP-Oleflex-Verfahren wird die für die endotherme Reaktion erforderliche Reaktionswärme über Zwischenheizer eingebracht, wobei häufig eine Serienanordnung von vier Zwischenheizern mit jeweils einem nachfolgenden adiabat betriebenen Reaktor vorgesehen ist. Nachteilig ist hierbei insbesondere der erhöhte Investitionsaufwand für die Zwischenheizer; darüber hinaus ist bei der absatzweisen indirekten Wärmeübertragung einer höherer Energieaufwand und eine Aufheizung des Einsatzgasstromes auf höhere Temperaturen, mit entsprechend verstärkt ablaufenden Nebenreaktionen gegenüber einer direkten Wärmeübertragung erforderlich.

Eine direkte Wärmeübertragung realisiert das autotherme BASF-Verfahren, bei dem für die Bereitstellung der für die endotherme Reaktion erforderlichen Wärme ein Teil des paraffinhaltigen Einsatzgasstromes und darüber hinaus ein Teil des bei der Oxidehydrierung entstehenden Wasserstoffes verbrannt wird. Vorteilhaft ist die bessere energetische Nutzung und darüber hinaus, dass bei der direkten Wärmeübertragung niedrigere Temperaturen, mit entsprechend geringerer Belastung des Einsatzgasstromes, erforderlich sind. Für das autotherme BASF-Verfahren sind in der Regel zwei Festbettreaktoren erforderlich, die abwechselnd im Oxidehydrier- bzw. Regenerierbetrieb gefahren werden, mit entsprechendem Investitionsaufwand.

Dokument DE-A 10 2004 032 129 beschreibt ein Verfahren zur Oxidehydrierung, und zwar zur Herstellung von Acrolein, Acrylsäure oder deren Gemisch aus Propan, bei dem man Propan zunächst heterogen katalysiert zu Propenen dehydriert, Nebenkomponenten abtrennt und das verbleibende Propan und Propen enthaltene Gasgemisch der heterogen katalysierten Partialoxidation zu Acrolein oder Acrylsäure oder deren Gemisch als Zielprodukt unterwirft. Der Sauerstoff wird stufenweise zugeführt und In einem statischen Mischer mit dem Produktgasstrom gemischt.

Das Dokument beschreibt insbesondere nicht eine Einmischvorrichtung zum Einsatz in einem Reaktor zur Durchführung einer kontinuierlichen Oxidehydrierung mit zwei oder drei hintereinander angeordneten Reihen von Rohren mit Turbulenzerzeugern auf deren Außenseite sowie einem den Rohren vorgeschalteten Lochblech und einem den Rohren nachgeschalteten Lochbleche.

Es war demgegenüber Aufgabe der Erfindung, einen Reaktor und ein Verfahren zur Durchführung von Oxidehydrierungen gesättigter Kohlenwasserstoffe zur Verfügung zu stellen, das die Vorteile bekannter Reaktoren und Verfahren verbindet, insbesondere die Vorteile des Katalysatorwanderbettes nach dem UOP-Oleflex-Verfahren und die energetischen Vorteile des autothermen BASF-Verfahrens.

Entsprechend wurde ein Reaktor zur Durchführung einer kontinuierlichen Oxidehydrierung eines Einsatzgastromes von gesättigten Kohlenwasserstoffen nach Vormischen mit einem Sauerstoff enthaltenden Gasstrom an einem Katalysatorwanderbett, das in Reaktorlängsrichtung zwischen zwei konzentrischen zylindrischen Halteeinrichtungen unter Freilassung eines zentralen Innenraumes und eines Zwischenraumes zwischen dem Katalysatorwanderbett und dem Reaktorinnenmantel eingebracht ist, unter Erhalt eines Reaktionsgasgemisches, der dadurch gekennzeichnet ist, dass
der Reaktor zwei oder mehrere Reaktorabschnitte aufweist, die voneinander mit alternierend im zentralen Innenraum angeordneten scheibenförmigen Umlenkblechen und im Zwischenraum zwischen dem Katalysatorwanderbett und dem Reaktorinnenmantel angeordneten ringförmigen Umlenkblechen in Tellberelchen getrennt sind, mit jeweils einer in Strömungsrichtung des Reaktionsgasgemisches vor dem Katalysatorwanderbett angeordneten Einmischvorrichtung, die aus den folgenden Elementen gebildet ist:
- zwei oder drei hintereinander angeordnete Reihen von Rohren mit Turbulenzerzeugern auf der Außenseite derselben, die Rippenrohre sind, mit einem Außendurchmesser der Rohre im Bereich zwischen 25 und 50 mm, wobei die zweite Reihe der Rippenrohre auf Lücke zur ersten Reihe der Rippenrohre angeordnet ist sowie für den Fall von drei Reihen von Rippenrohren, die dritte Reihe von Rippenrohren auf Lücke zur zweiten Reihe der Rippenrohre angeordnet ist, und die den Durchtrittsquerschnitt für den Einsatzgasstrom auf 1/2 bis 1/10 des freien Durchtrittsquerschnitts verengen, wobei durch die Innenräume der Rohre der sauerstoffhaltige Gasstrom geleitet und über Öffnungen der Rohre in den Einsatzgasstrom eingedüst wird, und wobei die Öffnungen in den Rippengängen zwischen den Rippen der Rippenrohre angeordnet sind, mit jeweils zwei Öffnungen pro Rippengang zwischen den Rippen der Rippenrohre, an diametral entgegengesetzten Stellen der Rippengänge, mit dem geringsten Abstand zu dem jeweils benachbarten Rippenrohr in der Rippenrohrreihe.sowie mit
- einem den Rohren vorgeschalteten Lochblech und einem den Rohren nachgeschalteten Lochblech
wobei das zweite, nachgeschaltete Lochblech Öffnungen aufweist, deren Durchmesser größer oder gleich dem Durchmesser des vorgeschalteten Lochbleches ist,
und wobei die Einmischvorrichtung eine Bautiefe, d. h. einen Abstand zwischen dem vorgeschalteten Lochblech und dem nachgeschalteten Lochblech , im Bereich zwischen 100 und 200 mm aufweist.

Es wurde gefunden, dass durch Verwendung einer speziellen Einmischvorrichtung die eine weitgehend homogene Vermischung bei sehr kurzen Verzögerungszeiten, unter 150 ms oder auch unter 50 ms, gewährleistet eine kontinuierliche Zuführung eines Sauerstoff enthaltenden Gases in maßgeschneiderten Mengen entsprechend dem Reaktionsfortschritt an einem Katalysatorwanderbett möglich ist.

In der Einmischvorrichtung werden der Einsatzgasstrom von gesättigten Kohlenwasserstoffen und der Sauerstoff enthaltende Gasstrom vorgemischt. Unter Vormischen wird vorliegend ein Mischen vor Eintritt in das Katalysatorwanderbett verstanden.

Durch den Einsatz von als Wärmetauscher bekannten und handelsüblichen Rippenrohren durch geringfügige Modifizierung derselben, indem Öffnungen in den Rippengängen zwischen den Rippen vorgesehen werden, werden die Zwischenräume der Rippengänge zwischen den Rippen als nahezu ideale Mischkammern mit hoher Turbulenz genutzt, indem der Einsatzgasstrom im Wesentlichen quer zu den Rippenrohren und ein Sauerstoff enthaltender Gasstrom durch die Innenräume der Rippenrohre über die Öffnungen in den Rippengängen in den Einsatzgasstrom eingedüst wird.

Die Volumenströme des Einsatzgasstromes und des Sauerstoff enthaltenden Gasstromes sind in der Regel stark unterschiedlich, was die Mischaufgabe entsprechend erschwert: der Mengenstrom des Sauerstoff enthaltenden Gases kann insbesondere zwischen 5 und 30 % des Mengenstromes des Einsatzgasstromes betragen.

Der Katalysator ist in einem vertikal aufrecht stehenden, in der Regel zylindrischen, Reaktor, zwischen zwei konzentrischen zylindrischen Halteeinrichtungen, die in einer bevorzugten Ausführungsform Kantenspaltsiebe sein können, angeordnet und die von oben, insbesondere über einen Vorratsbehälter, befüllt und im unteren Bereich über geeignete Einrichtungen entleert werden. Die rieselförmigen Katalysatorformkörper gleiten im Reaktor zwischen den zylindrischen Halteeinrichtungen, über die gesamte Höhe des Reaktors von oben nach unten in durchgehenden, engen Spalten. Die maximalen linearen Abmessungen der Spalte sind bevorzugt kleiner oder gleich der Hälfte des kleinsten Außendurchmessers der Katalysatorformkörper und liegen bevorzugt im Bereich zwischen 0,75 und 2,00 mm, insbesondere bevorzugt etwa 1,2 mm.

Zwischen den einzelnen Reaktorabschnitten befinden sich im Katalysatorwanderbett kurze Abdichtstrecken, deren Länge insbesondere etwa einer Bettstärke entspricht, um Bypässe des Reaktionsgasgemisches weitgehend zu unterdrücken.

Ein bevorzugter Katalysator, der selektiv die Verbrennung von Wasserstoff katalysiert, enthält Oxide und/oder Phosphate, ausgewählt aus der Gruppe bestehend aus den Oxiden und/oder Phosphaten von Germanium, Zinn, Blei, Arsen, Antimon oder Bismut. Ein weiterer bevorzugter Katalysator, der die Verbrennung von Wasserstoff katalysiert, enthält ein Edelmetall der VIII. und/oder I. Nebengruppe.

Die eingesetzten Dehydrierungskatalysatoren weisen im Allgemeinen einen Träger und eine Aktivmasse auf. Der Träger besteht dabei in der Regel aus einem wärmebeständigen Oxid oder Mischoxid. Bevorzugt enthalten die Dehydrierungskatalysatoren ein Metalloxid, das ausgewählt ist aus der Gruppe bestehend aus Zirkondioxid, Zinkoxid, Aluminiumoxid, Siliciumdioxid, Titandioxid, Magnesiumoxid, Lanthanoxid, Ceroxid und deren Gemischen, als Träger. Bei den Gemischen kann es sich um physikalische Mischungen oder auch um chemische Mischphasen wie Magnesium- oder Zinkaluminiumoxid-Mischoxide handeln. Bevorzugte Träger sind Zirkondioxid und/oder Siliziumdioxid, besonders bevorzugt sind Gemische aus Zirkondioxid und Siliziumdioxid.

Die Aktivmasse der Dehydrierungskatalysatoren enthalten im allgemeinen ein oder mehrere Elemente der VIII. Nebengruppe, bevorzugt Platin und/oder Palladium, besonders bevorzugt Platin. Darüber hinaus können die Dehydrierungskatalysatoren ein oder mehrere Elemente der I. und/oder II. Hauptgruppe aufweisen, bevorzugt Kalium und/oder Cäsium. Weiterhin können die Dehydrierungskatalysatoren ein oder mehrere Elemente der III. Nebengruppe einschließlich der Lanthaniden und Actiniden enthalten, bevorzugt Lanthan und/oder Cer. Schließlich können die Dehydrierungskatalysatoren ein oder mehrere Elemente der III. und/oder IV. Hauptgruppe aufweisen, bevorzugt ein oder mehrere Elemente aus der Gruppe bestehend aus Bor, Gallium, Silizium, Germanium, Zinn und Blei, besonders bevorzugt Zinn.

In einer bevorzugten Ausführungsform enthält der Dehydrierungskatalysator mindestens ein Element der VIII. Nebengruppe, mindestens ein Element der I. und/oder II. Hauptgruppe, mindestens ein Element der III. und/oder IV. Hauptgruppe und mindestens ein Element der III. Nebengruppe einschließlich der Lanthaniden und Actiniden.

Beispielsweise können erfindungsgemäß alle Dehydrierkatalysatoren eingesetzt werden, die in den WO 99/46039, US 4,788,371, EP-A 705 136, WO 99/29420, US 5,220,091, US 5,430,220, US 5,877,369, EP 0 117 146, DE-A 199 37 106, DE-A 199 37 105 und DE-A 199 37 107 offenbart werden. Besonders bevorzugte Katalysatoren für die vorstehend beschriebenen Varianten der autothermen Propan-Dehydrierung sind die Katalysatoren gemäß den Beispiel 1, 2, 3 und 4 der DE-A 199 37107.

Bevorzugt werden pneumatisch förderbare und wasserdampffeste Katalysatoren eingesetzt.

Die autotherme Propan-Dehydrierung wird bevorzugt in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit der Katalysatoren erhöht wird. Dabei werden die organischen Ablagerungen in Kohlenmonoxid, Kohlendioxid und gegebenenfalls Wasser umgewandelt.

Frischer und/oder regenerierter Katalysator wird von oben, insbesondere über den Vorratsbehälter aufgegeben und am unteren Ende des Reaktors, beladen mit Schadprodukten, abgezogen. Der Katalysator wandert somit von oben nach unten durch den Reaktor. Der beladene Katalysator wird insbesondere pneumatisch in einen separaten Regenerierturm befördert und dort mit sauerstoffhaltigern Gas in bekannter Weise, durch Entspannung, Spülen, Abbrennen, Rekristallisieren, Niederpressen und Zuschalten regeneriert und anschließend, ebenfalls pneumatisch erneut auf den Kopf des Reaktors zurückgeführt.

Das Reaktionsgasgemisch strömt das Katalysatorwanderfestbett von der Seite einer Anströmfläche desselben an und verlässt das Katalysatorwanderbett über eine Abströmfläche.

Rippenrohre sind in der chemischen Verfahrenstechnik bekannt und werden insbesondere als Wärmetauscherrohre eingesetzt. Rippenrohre und ihre Herstellung sind beispielsweise in DE-A 1 950 246 oder DE-A 2 131 085, beschrieben.

Ein Rippenrohr ist aus einem Rohr, in der Regel einem Metallrohr, mit zylindrischem Außenumfang gebildet, mit auf diesem, in der Regel durch Schweißen entlang einer Längskante desselben aufgebrachten länglichen Streifens, den Rippen. Die Rippen sind häufig spiral- bzw. wendelförmig auf dem Außenumfang des Rohres, können aber auch in Längsrichtung desselben angebracht sein. Sie haben normalerweise eine glatte durchlaufende Oberfläche, können aber auch perforiert sein. Sie können durchlaufend, aber auch, vorteilhaft, unter Ausbildung von Segmenten bis auf eine Rippenbasis eingeschnitten sein. Die Segmente können hierbei unterschiedliche Geometrien aufweisen, beispielsweise in Form von Rechtecken, Trapezen, usw.. Die Einschnitte zwischen den Segmenten können mit oder ohne Materialverlust ausgeführt sein. Besonders vorteilhaft können die Segmente gegenüber der Rippenbasis in einem Winkel verdreht bzw. geschränkt ausgebildet sein, um über einen Anstellwinkel die Turbulenz insbesondere in den Bereichen zwischen den Rippen, den Rippengängen, zu erhöhen, und entsprechend die Mischwirkung zu verbessern.

Eine dichte Anordnung von Rippen über die Rohrlänge ist vorteilhaft, insbesondere können 100 bis 300 Umläufe der Rippen pro Meter Rohrlänge vorgesehen sein.

Es werden Rohre mit einem Außendurchmesser im Bereich zwischen 26 und 50 mm, eingesetzt.

Die Rippenhöhe bezogen auf den Außendurchmesser der Rohre liegt vorteilhaft in einem Bereich zwischen 1/10 und 1/2.

Die Rippendicke kann vorteilhaft zwischen 0,3 und 1,5 mm liegen.

Bei eingeschnittenen Rippen ist es vorteilhaft, Segmente mit einer Breite zwischen 3 und 12 mm, bevorzugt zwischen 4 und 8 mm, auszubilden.

Die Rohre können jeden Querschnitt aufweisen, beispielsweise kreisförmig, oval oder auch polygonal, zum Beispiel dreieckig.

Die Rippenrohre sind in Reihen, parallel zueinander angeordnet, wobei eine Rippenrohrreihe entlang eines Kreisradius angeordnet ist.

Bei dem vorliegenden Radialstromreaktor, mit radialer Strömungsrichtung des Reaktionsgasgemisches, ist das Katalysatorwanderbett in Form eines Hohlzylinders mit der Bettdicke entsprechender Wandstärke, in geeigneten Aufnahmevorrichtungen, wie vorstehend beschrieben, angeordnet. Auf der Anströmseite des Katalysatorwanderbettes, die alternierend innen oder außen ist, sind die Rippenrohre entlang eines zum Katalysatorwanderbett konzentrischen Kreisringes angeordnet.

Es hat sich gezeigt, dass für die erfindungsgemäße Mischaufgabe zwei oder drei Reihen von Rippenrohren geeignet sind.

In einer bevorzugten Ausführungsform kann die Zusammensetzung des sauerstoffhaltigen Gasstromes in den einzelnen Reihen von Rohren mit Turbulenzerzeugern untereinander unterschiedlich sein. Insbesondere kann in die erste Reihe von Rohren mit Turbulenzerzeugern ein Sauerstoff enthaltender Gasstrom mit einer anderen Zusammensetzung gegenüber der zweiten Reihe von Rohren mit Turbulenzerzeugern eingeleitet werden.

Die zweite Reihe von Rippenrohren ist gegenüber der ersten auf Lücke angeordnet sowie, für den Fall von drei Rippenrohrreihen, die dritte Rippenrohrreihe auf Lücke zur zweiten Rippenrohrreihe. Die zweite und gegebenenfalls auch die dritte Reihe von Rippenrohren können vorteilhaft von einem Wärmeträger durchströmt sein. Es ist auch möglich, die zweite und gegebenenfalls auch die dritte Reihe von Rippenrohren aus Vollmaterial beliebigen Querschnitts auszubilden.

Innerhalb einer Rippenrohrreihe sollen Rippenrohre gleicher Geometrie eingesetzt werden, diese kann jedoch innerhalb der Rippenrohrreihen variieren.

Die Rippenrohre weisen am Außenumfang der sie bildenden Rohre in den Rippengängen zwischen den Rippen pro Rippengang jeweils zwei diametral entgegengesetzte Öffnungen auf, an den Stellen mit dem geringsten Abstand zu dem jeweils benachbarten Rippenrohr in der Rippenrohrreihe. Durch diese Öffnungen wird der Sauerstoff enthaltende Gasstrom in die Rippengänge zwischen den Rippen in den Einsatzgasstrom eingedüst. Es werden somit in den Rippengänge eine Vielzahl von feinskaligen Mischkammern zur Verfügung gestellt, mit hoher Turbulenz, insbesondere bei segmentartig eingeschnittenen Rippen, wobei dieser Effekt durch verschränkte Anstellung der Rippensegmente noch erhöht werden kann. Dadurch wird eine ausgezeichnete Mischgüte im Mikrobereich erreicht.

Im Innenraum der Rippenrohre kann vorteilhaft jeweils ein konzentrisches Einsteckrohr mit in geeigneten Abständen am Außenumfang desselben, bevorzugt jeweils zwei diametral entgegengesetzt angeordneten Ausströmöffnungen, vorgesehen sein, um die Vorverteilung des Sauerstoff enthaltenden Gasstromes über die Rohrlänge und damit auch einen weitgehenden Temperaturausgleich desselben zu gewährleisten.

Bevorzugt wird der Sauerstoff enthaltende Gasstrom in die Rippenrohre über eine Ringleitung als Hauptverteiler, und besonders bevorzugt über zwei Ringleitungen, an jedem Ende der Rippenrohre, gleichmäßig aufgegeben.

Den Rippenrohrreihen ist, ebenfalls quer zur Anströmrichtung des Sauerstoff enthaltenden Gasstromes und somit im Wesentlichen auf einem konzentrischen Kreisring zu den Rippenrohrreihen, ein Lochblech vorgeschaltet.

Das vorgeschaltete Lochblech weist Öffnungen auf, deren Gesamtfläche bevorzugt bezogen auf die Querschnittsfläche der Zuführung des Sauerstoff enthaltenden Gasstromes kleiner oder gleich 0,5, insbesondere kleiner oder gleich 0,3, ist.

Das vorgeschaltete Lochblech ist vorteilhaft zur Anströmfläche der ersten Rippenrohrreihe um das Sieben- bis Zwanzigfache des Durchmessers der Öffnungen im vorgeschalteten Lochblech beabstandet.

Der Durchmesser der Öffnungen im vorgeschalteten Lochblech ist vorteilhaft kleiner als die Hälfte des lichten Abstandes der Rippen zwischen zwei aufeinander folgenden Umläufen.

Die Einmischvorrichtung weist in Abströmrichtung aus derselben ein zweites, nachgeschaltetes Lochblech auf, mit Öffnungen, deren Durchmesser größer oder gleich dem Durchmesser des vorgeschalteten Lochbleches ist.

Die Blechdicke beider Lochbleche, des vorgeschalteten und des nachgeschalteten Lochbleches, bezogen auf den Durchmesser der Öffnungen in den Lochblechen liegt bevorzugt im Bereich zwischen 0,75 und 2,0.

Das nachgeschaltete Lochblech ist vorteilhaft von der Abströmebene der letzten Reihe von Rippenrohren um das 0,75 bis Zweifache des Durchmesser der Rippenrohre der letzten der Rippenrohrreihe beabstandet angeordnet.

Das nachgeschaltete Lochblech ist vorteilhaft zum Eintritt in das Katalysatorwanderbett in einem Abstand zwischen 5 und 20 mal dem Durchmesser der Öffnungen in demselben beabstandet.

Der Werkstoff für alle Reaktorbereiche, die mit dem Reaktionsgasgemisch in Kontakt kommen, insbesondere für die Rippenrohre und die Lochbleche wie auch den Reaktorinnenmantel ist bevorzugt nichtaufhohlender Stahl.

Die Einmischvorrichtung ist im Wesentlichen quer zur Strömungsrichtung des Sauerstoff enthaltenden Gasstromes angeordnet. Darunter wird verstanden, dass der Sauerstoff enthaltende Gasstrom senkrecht zur Hauptfläche der Einmischvorrichtung zugeführt wird, die bei Radiälstromreaktoren gekrümmt ist. Unter im Wesentlichen quer sollen jedoch auch Abweichungen von der Senkrechten zwischen 5° oder ± 10°, oder auch ± 30°, verstanden werden.

Die Einmischvorrichtung kann mit Bautiefen, das heißt einem Abstand zwischen dem vorgeschalteten und dem nachgeschalteten Lochblech im Bereich zwischen 100 und 200 mm, eine ausgezeichnete, nahezu 100 %ige Mischgüte erreichen, bei einem Druckverlust für den Einsatzgasstrom in der Größenordnung von 20 mbar und einem Druckverlust für den Sauerstoff enthaltenden Strom, der bereits aus Sicherheitsgründen unter einem gewissen Überdruck stehen muss, im Bereich von etwa 50 bis 100 mbar.

Es wird eine extrem hohe Anzahl von Eindüsstellen des Einsatzgasstromes in den Sauerstoff einthaltenden Gasstrom, in der Größenordnung von 10000 Eindüsstellen pro m², er-reicht.

Gegenstand, der Erfindung ist auch ein Verfahren zur Durchführung von kontinuierlichen Oxidehydrierungen in einem vorstehend beschriebenen Reaktor.

Der Einsatzgasstrom von gesättigten Kohlenwasserstoffen kann insbesondere ein Propan oder ein Butan enthaltender Gasstrom sein.

In den Einsatzgasstrom wird kontinuierlich über die Einmischvorrichtung ein Sauerstoff enthaltender Gasstrom, insbesondere Luft oder technisch reiner Sauerstoff, eingedüst. Dabei entsteht am Ende des ersten Reaktorabschnittes ein Reaktionsgasgemisch, das insbesondere, neben nicht umgesetzten gesättigten Kohlenwasserstoffen, die entsprechenden Olefine sowie Wasserstoff enthält, und das in den nächsten Reaktorabschnitt als Einsatzgasstrom einströmt, wo kontinuierlich ein weiterer Sauerstoff enthaltender Gasstrom eingedüst wird. Diese Verfahrensführung wird wiederholt, bis das Reaktionsgasgemisch den letzten Reaktorabschnitt verlässt. Hierbei ist auch nach dem letzten Reaktorabschnitt der Umsatz der gesättigten Kohlenwasserstoffe nicht vollständig, sondern beispielsweise im Bereich von etwa 30 %.

In einer besonderen Ausführung wird ein Teilstrom des Reaktionsgasgemisches mit frischem, gesättigte Kohlenwasserstoffe enthaltenden Einsatzgasstrom, gemischt und in den Reaktor, in den ersten Reaktorabschnitt, recycliert. Der übrige Teilstrom des Reaktionsgasgemisches wird als Produktstrom abgezogen.

In einer weiteren bevorzugten Ausführungsform kann das den letzten Reaktorabschnitt verlassende Reaktionsgasgemisch zur Wärmeintegration benutzt werden, indem es den Einsatzgasstrom, enthaltend gesättigte Kohlenwasserstoffe, durch indirekten Wärmetausch vorwärmt.

Der Reaktor und das Verfahren kennzeichnen sich insbesondere durch eine vollkontinuierliche Strömungsführung des Reaktionsgasgemisches wodurch eine einheitliche, konstante Produktzusammensetzung über die Zeit erreicht wird.

Die Regenerierung des Katalysators erfolgt ebenfalls kontinuierlich, in einer kleinen, speziell dafür ausgelegten Apparatur und ist wesentlich einfacher gegenüber der Regenerierung des Katalysators im Verfahren nach dem Stand der Technik, mit Regenerierung des verbrauchten Katalysators im Oxidehydrierreaktor, wofür eine komplizierte Folge von Regenerierschritten, mit Umschaltung, Entspannung, Spülung, Abbrennen, Inertisieren, Wiederaufpressen und Umschalten erforderlich ist und entsprechend den Katalysator extrem schädigenden Temperatur- und Druckwechseln in kurzer Folge.

Durch die erfindungsgemäße Verfahrensweise wird weitgehend ein Investitionsvorteil in der Größenordnung von 40 % und eine gleichmäßige Zusammensetzung des abgezogenen Produktstromes erreicht.

Die Erfindung wird im Folgenden anhand einer Zeichnung näher erläutert. Es zeigen im Einzelnen:
- Figur 1: einen kreissegmentartigen Ausschnitt durch einen erfindungsgemäßen Reaktor mit Strömungsrichtung des Einsatzgasstromes von außen nach innen,
- Figur 2A: die Detaildarstellung eines Rippenrohres, mit Darstellung einer einzelnen Rippe und der Arbeitsgänge zu ihrer Ausbildung in Figur 2B sowie eine Querschnittsdarstellung durch ein Rippenrohr in Figur 2C,
- Figur 3: eine perspektivische Darstellung eines Rippenrohres,
- Figur 4A: eine Längsschnittdarstellung durch eine bevorzugte Ausführungsform für ein Rippenrohr mit Querschnittsdarstellung in Figur 4B,
- Figur 5: einen Längsschnitt durch eine Ausführungsform eines erfindungsgemäßen Reaktors,
- Figur 6: einen Längsschnitt durch eine weitere Ausführungsform eines erfindungsgemäßen Reaktors, mit Rückführung eines Teilstroms des Produktgasstromes und
- Figur 7: einen Längsschnitt durch eine weitere Ausführungsform eines erfindungsgemäßen Reaktors mit Vorerwärmung des Einsatzgasstromes durch den Produktgasstrom.

In den Figuren bezeichnen gleiche Bezugszeichen jeweils gleiche oder entsprechende Bauteile.

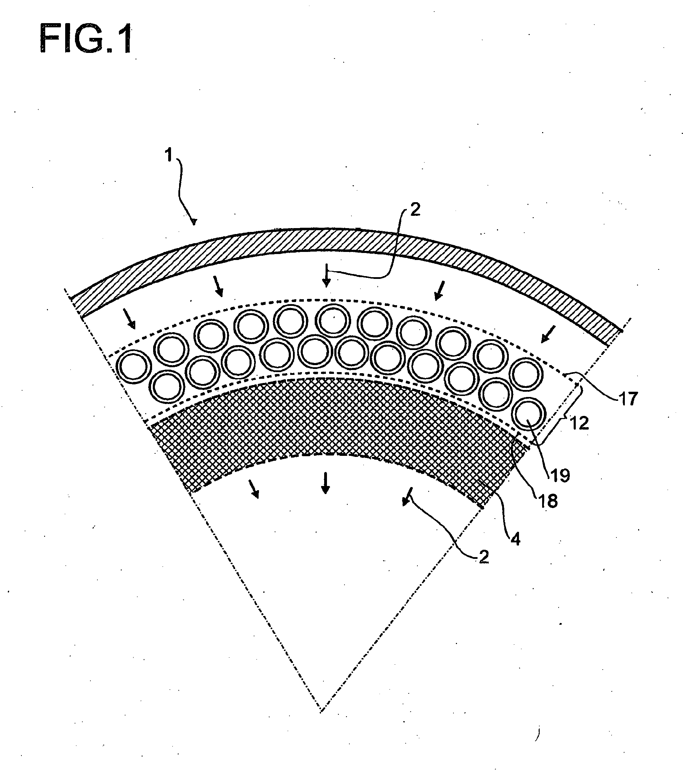

Figur 1 zeigt ein Kreissegment aus einem Querschnitt durch eine erste Ausführungsform eines erfindungsgemäßen Reaktors 1 mit Zuführung eines Einsatzgasstromes 2 am Reaktoraußenmantel und Abströmung desselben über den Innenraum des Reaktors. Der Einsatzgasstrom 2 trifft senkrecht auf eine Einmischvorrichtung 12, umfassend zwei Reihen von Rippenrohren 19, die auf Lücke angeordnet sind und der in Strömungsrichtung ein erstes Lochblech 17 vorgeschaltet und ein zweites Lochblech 18 nachgeschaltet ist. Die beiden Reihen der Rippenrohre 19 sowie das vorgeschaltete Lochblech 17 und das nachgeschaltete Lochblech 18 sind jeweils auf konzentrischen Kreisringen angeordnet. Das in der Einmischvorrichtung 12 vorgemischte Reaktionsgasgemisch durchströmt anschließend das Katalysatorwanderbett 4.

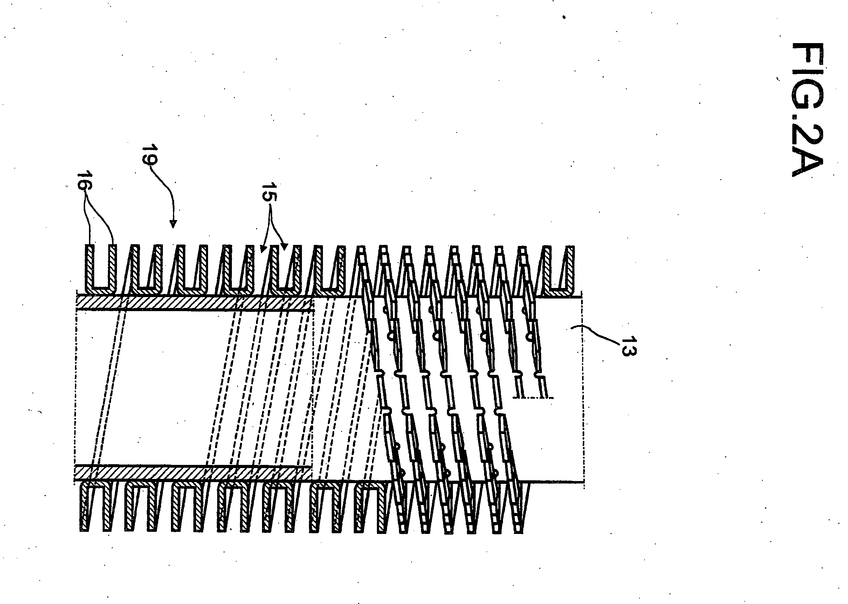

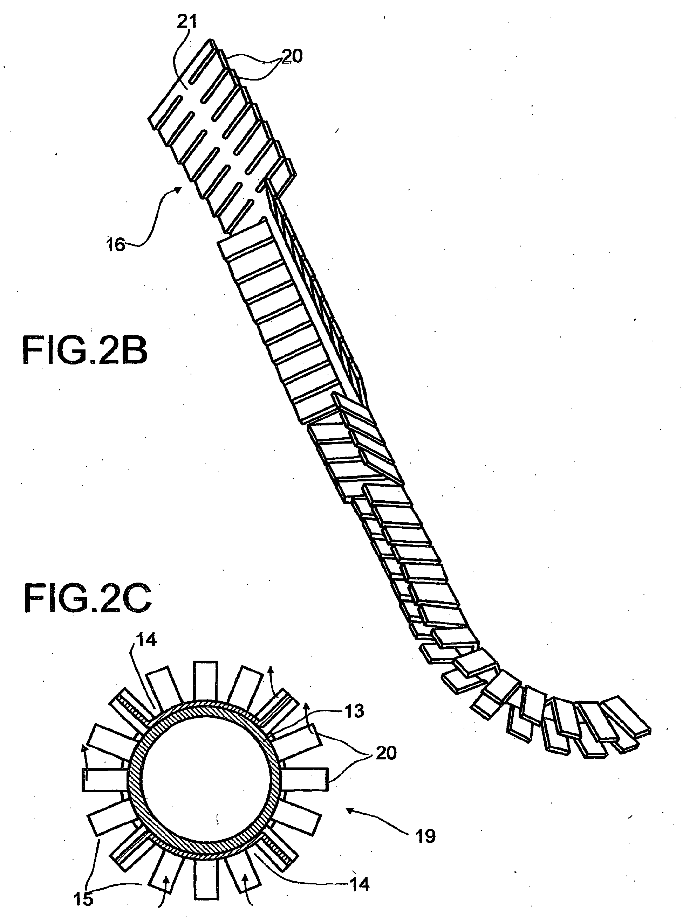

Die Figuren 2A bis 2C zeigen Detaildarstellungen von Rippenrohren 19 mit diametral gegenüber angeordneten Öffnungen 14 in den Rippengängen 15 zwischen den Rippen 16 der Rippenrohre 19. Hiervon zeigt Figur 2B eine Rippe 16, die bis auf eine Rippenbasis 21 durch Einschnitte in Segmente 20 aufgeteilt ist und Figur 2C einen Querschnitt durch ein Rippenrohr 19 mit Rohr 13, Rippengängen 15, und Segmenten 20.

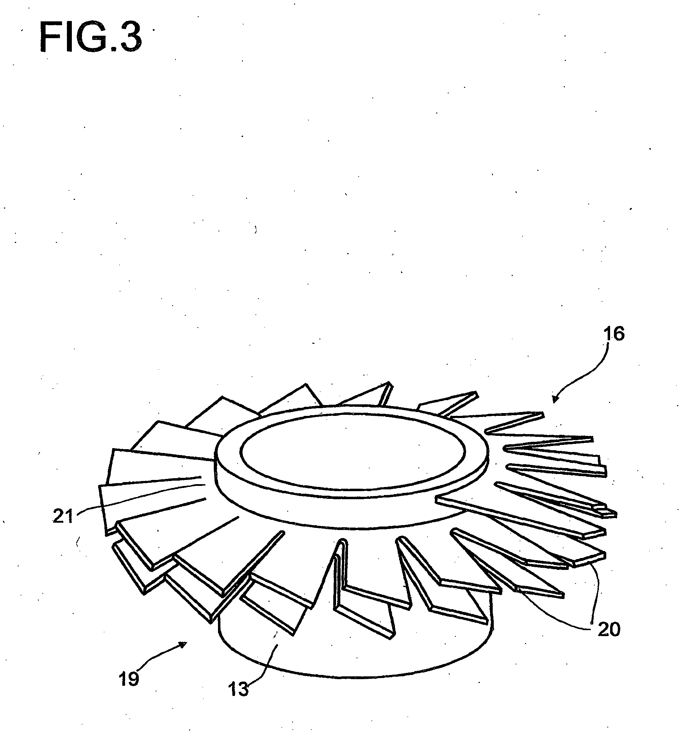

Figur 3 zeigt eine perspektivische Darstellung eines Rippenrohres 19 mit Rohr 13 und wendelförmig aufgebrachter Rippe 16, die in Segmente 20 mit Ausnahme einer durchgehenden Rippenbasis 21 aufgeteilt ist.

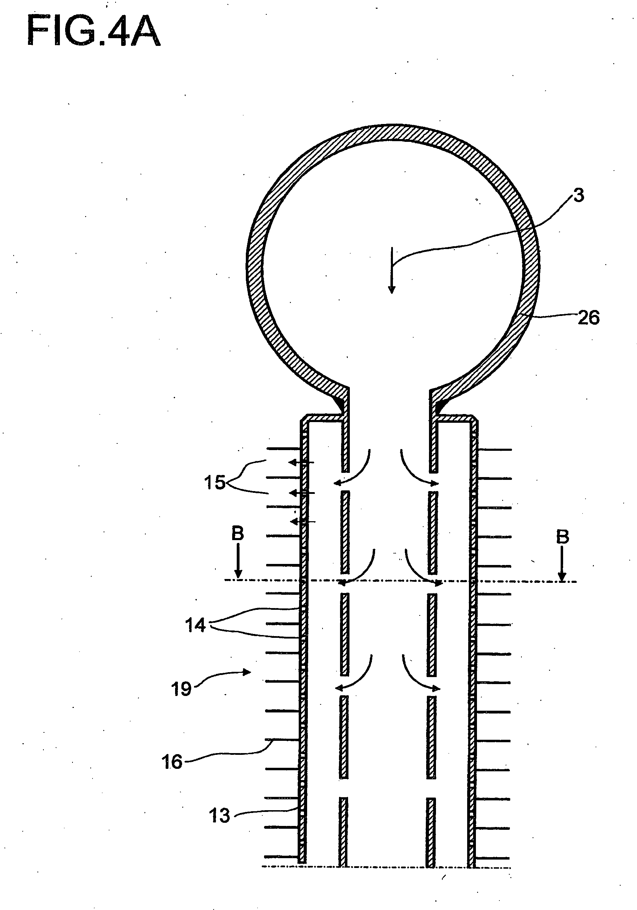

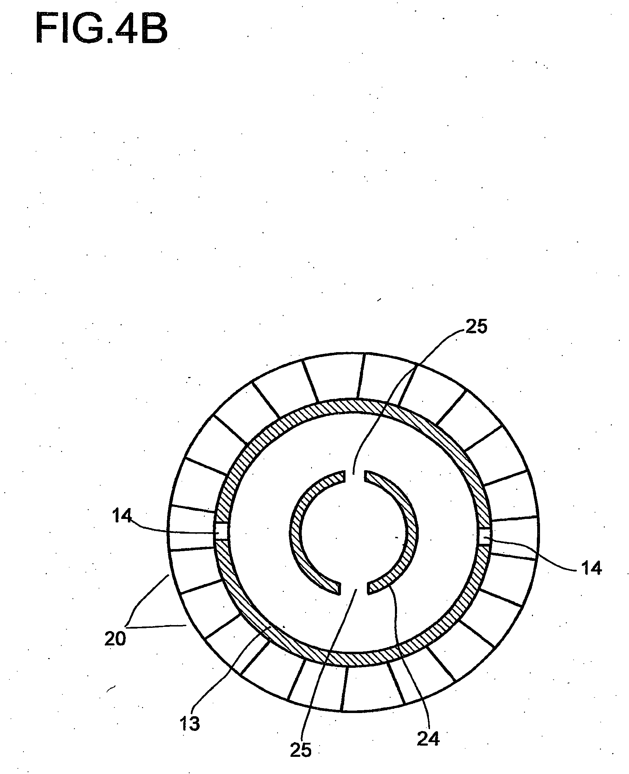

Figur 4A zeigt eine Längsschnittdarstellung durch ein Rippenrohr 19 mit Rohr 13 und Rippen 16, mit Öffnungen 14 in den Rippengängen 15 zwischen den Rippen 16 der Rippenrohre 19. Im Rohrinneren ist konzentrisch ein zentrales Einsteckrohr 24 vorgesehen, das über diametral entgegengesetzt angeordnete Öffnungen 25, die in der Querschnittdarstellung in der Ebene D-B in Figur 4B ersichtlich sind, den Sauerstoff enthaltenden Gasstrom 3 in Längsrichtung des Rippenrohres 13 verteilen. In Figur 4A ist ein Ende des Rippenrohres 19 mit Ringverteiler 26 für den Sauerstoff enthaltenden Gasstrom 3 auf die Rippenrohre 19 dargestellt.

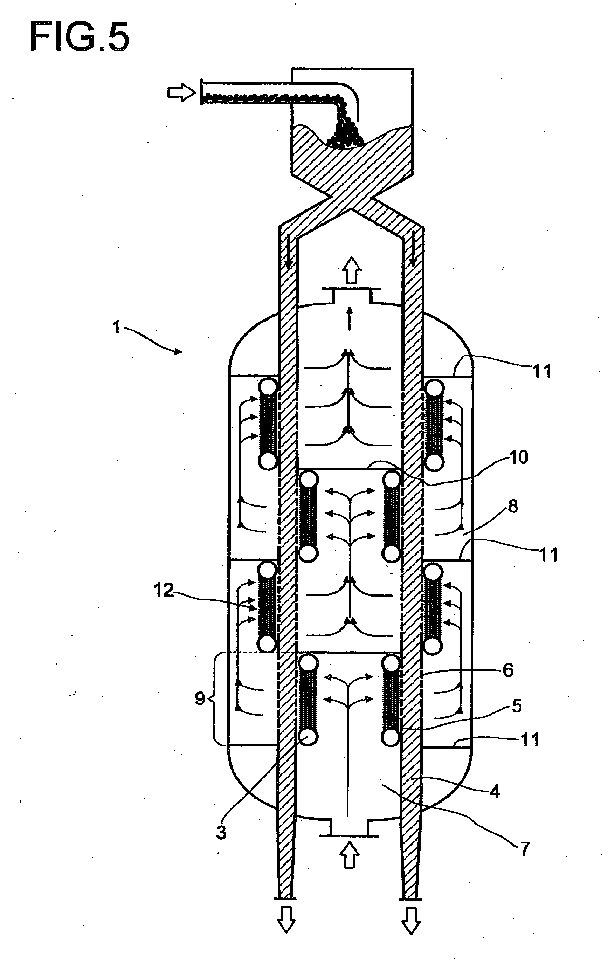

Figur 5 zeigt einen Längsschnitt durch eine Ausführungsform eines erfindungsgemäßen Reaktors 1 mit vier übereinander angeordneten Reaktorabschnitten 9. Der Sauerstoff enthaltende Gasstrom 3 wird über den Innenraum der Rippenrohre 19 in den Einsatzgasstrom 2 eingedüst. Der gesättigte Kohlenwasserstoff enthaltende Einsatzgasstrom 2 wird in den zentralen Innenraum 7 des ersten Reaktorabschnittes 9 geführt, und strömt über eine zylindrisch vor dem in konzentrisch zylindrischen Halteeinrichtungen 5 und 6 angebrachten Wanderbettkatalysator 4 über eine Einmischvorrichtung 12 mit Rippenrohren 19 und diesem vorgeschalteten Lochbleche 17 sowie nachgeschaftetem Lochblech 18. Der erste Reaktorabschnitt 9 ist in Teilbereichen, und zwar im Bereich des zentralen Innenraumes 7 durch ein scheibenförmiges Umlenkblech 10 abgetrennt, dergestalt, dass das den ersten Reaktorabschnitt 9 verlassende Reaktionsgasgemisch über den Zwischenraum 8 am Reaktorinnenmantel in den darüber angeordneten, zweiten Reaktorabschnitt 9 strömt. Im zweiten Reaktorabschnitt 9 ist die Einmischvorrichtung 12 wiederum in Strömungsrichtung vor dem Katalysatorwanderbett 4, das heißt in diesem Fall konzentrisch außerhalb desselben, angeordnet. Der zweite Reaktorabschnitt 9 ist durch ringförmige Umlenkbleche 11 im

Bereich des Zwischenraumes 8 am Reaktorinnenmantel vom darauf folgenden, dritten Reaktorabschnitt 9 getrennt. Die Abfolge von scheibenförmigen Umlenkblechen 10 und ringförmigen Umlenkblechen 11 alterniert, dergestalt, dass der dritte Reaktorabschnitt 9 wiederum von einem scheibenförmigen Umlenkblech 10 nach oben abgetrennt ist und der vierte Reaktorabschnitt 9 entsprechend von einem ringförmigen Umlenkblech 11.

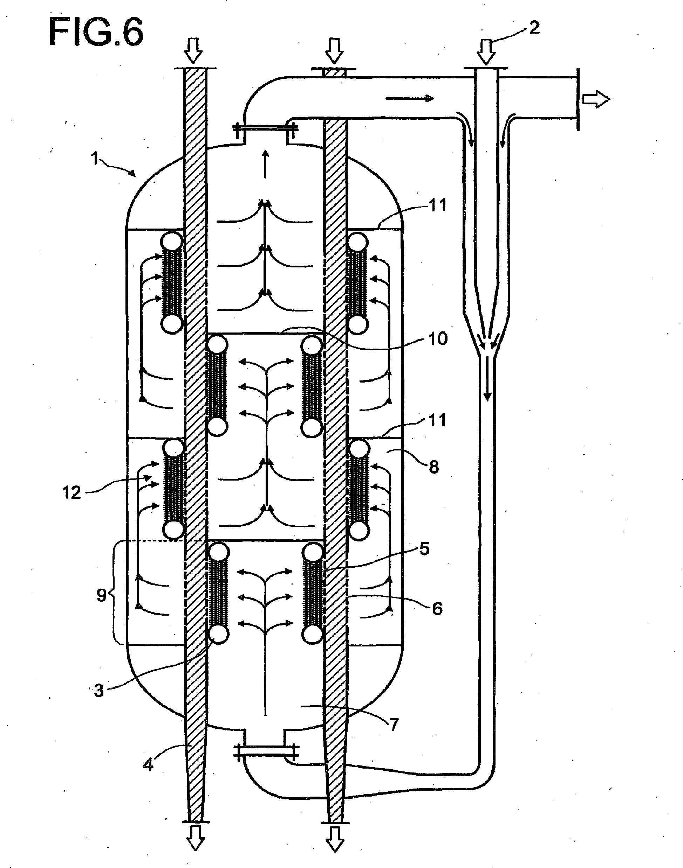

Figur 6 zeigt einen Längsschnitt durch eine weitere Ausführungsform eines erfindungsgemäßen Reaktors mit Rückführung eines Teilstromes des Produktgasstromes.

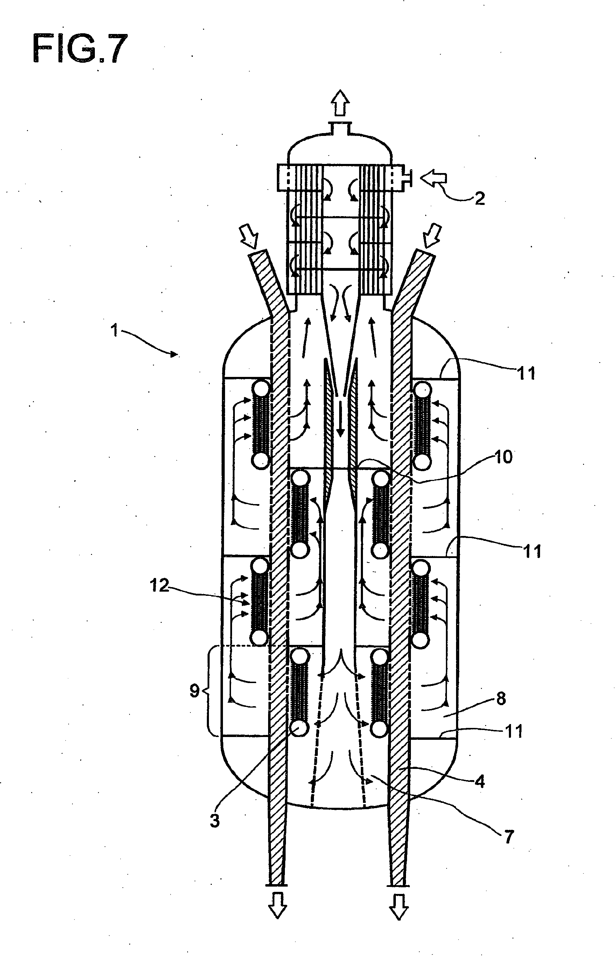

Die Ausführungsform in Figur 7 weist zusätzlich im oberen Reaktorbereich einen Wärmetauscher zur Wärmeintegration auf, wobei das heiße Produktgas den gesättigte Kohlenwasserstoffe enthaltenden Einsatzgasstrom 2 vorerwärmt.

### Bezugszeichenliste

- 1: Reaktor
- 2: Einsatzgasstrom
- 3: Sauerstoff enthaltender Gasstrom
- 4: Katalysatorwanderbett
- 5, 6: konzentrische zylindrische Halteeinrichtungen
- 7: zentraler Innenraum
- 8: Zwischenraum am Reaktorinnenmantel
- 9: Reaktorabschnitt
- 10: scheibenförmige Umlenkbleche
- 11: ringförmige Umlenkbleche
- 12: Einmischvorrichtung
- 13: Rohre
- 14: Öffnungen
- 15: Rippengänge
- 16: Rippen
- 17: vorgeschaltetes Lochblech
- 18: nachgeschaltetes Lochblech
- 19: Rippenrohre
- 20: Segmente
- 21: Rippenbasis
- 22: Öffnungen in 17
- 23: Öffnungen in 18
- 24: zentrales Einsteckrohr
- 25: Öffnungen in 24
- 26: Ringverteiler

## Patentansprüche

1. Reaktor (1) zur Durchführung einer kontinuierlichen Oxidehydrierung eines Einsatzgasstromes (2) von gesättigten Kohlenwasserstoffen nach Vormischen mit einem Sauerstoff enthaltenden Gasstrom (3) an einem Katalysatorwanderbett (4), das in Reaktorlängsrichtung zwischen zwei konzentrischen zylindrischen Halteeinrichtungen (5, 6) unter Freilassung eines zentralen innenraumes (7) und eines Zwischenraumes (8) zwischen dem Katalysatorwanderbett (4) und dem Reaktorinnenmantel eingebracht ist, unter Erhalt eines Reaktionsgasgemisches, **dadurch gekennzeichnet, dass**
der Reaktor (1) zwei oder mehrere Reaktorabschnitte (9) aufweist, die voneinander mit alternierend im zentralen Innenraum (7) angeordneten scheibenförmigen Umlenkblechen (10) und im Zwischenraum zwischen dem Katalysatorwanderbett (4) und dem Reaktorinnenmantel angeordneten ringförmigen Umlenkblechen (11) in Teilbereichen getrennt sind, mit jeweils einer in Strömungsrichtung des Reaktionsgasgemisches vor dem Katalysatorwanderbett (4) angeordneten Einmischvorrichtung (12), die aus den folgenden Elementen gebildet ist:
- zwei oder drei hintereinander angeordnete Reihen von Rohren (13) mit Turbulenzerzeugern auf der Außenseite derselben, die Rippenrohre (19) sind mit einem Außendurchmesser der Rohre im Bereich zwischen 25 und 50 mm, wobei die zweite Reihe der Rippenrohre (19) auf Lücke zur ersten Reihe der Rippenrohre (19) angeordnet ist sowie für den Fall von drei Reihen von Rippenrohren (19), die dritte Reihe von Rippenrohren (19) auf Lücke zur zweiten Reihe der Rippenrohre (19) angeordnet ist, und die den Durchtrittsquerschnitt für den Einsatzgasstrom (2) auf 1/2 bis 1/10 des freien Durchtrittsquerschnitts verengen, wobei durch die Innenräume der Rohre (13) der sauerstoffhaltige Gasstrom (3) geleitet und über Öffnungen (14) der Rohre (13) in den Einsatzgasstrom (2) eingedüst wird, und wobei die Öffnungen (14) in den Rippengängen (15) zwischen den Rippen (16) der Rippenrohre (19) angeordnet sind, mit jeweils zwei Öffnungen (14) pro Rippengang (15) zwischen den Rippen (16) der Rippenrohre (19), an diametral entgegengesetzten Stellen der Rippengänge (15), mit dem geringsten Abstand zu dem jeweils benachbarten Rippenrohr (19) in der Rippenrohrreihe.sowie mit einem den Rohren (13) vorgeschalteten Lochblech (17) und einem den Rohren (13) nachgeschalteten Lochblech (18)
wobei das zweite, nachgeschaltete Lochblech (18) Öffnungen (14) aufweist, deren Durchmesser größer oder gleich dem Durchmesser des vorgeschalteten Lochbleches (17) ist,
und wobei die Einmischvorrichtung (5) eine Bautiefe, d. h. einen Abstand zwischen dem vorgeschalteten Lochblech (17) und dem nachgeschalteten Lochblech (18), im Bereich zwischen 100 und 200 mm aufweist.

2. Reaktor nach Anspruch 1, **dadurch gekennzeichnet, dass** zwei bis acht Reaktorabschnitte (9), bevorzugt vier Reaktorabschnitte (9), vorgesehen sind.

3. Reaktor (1) nach Anspruch 1 oder 2, **dadurch gekennzeichnet, dass** die Rippenrohre (19) den freien Durchtrittsquerschnitt für den Einsatzgasstrom (2) auf 1/3 bis 1/6 desselben verengen.

4. Reaktor (1) nach einem der Ansprüche 1 bis 3, **dadurch gekennzeichnet, dass** die Rippenrohre (19) aus Rohren (19) mit zylindrischem Außenumfang gebildet sind, mit spiralförmig auf diesem entlang einer Streifen-Längskante aufgeschweißten, aus länglichen Streifen bestehenden Rippen (16), die unter Ausbildung von Segmenten (20) mit Ausnahme einer Rippenbasis (21) eingeschnitten sind.

5. Reaktor (1) nach Anspruch 4, **dadurch gekennzeichnet, dass** die Segmente (20) gegenüber der Rippenbasis (21) in einem Winkel hierzu verdreht sind.

6. Reaktor (1) nach einem der Ansprüche 1 bis 5, **dadurch gekennzeichnet, dass** die Rippenrohre (19) 100 bis 300 Umläufe der Rippen (9) pro Meter Länge des Rippenrohres (19) aufweisen.

7. Reaktor (1) nach einem der Ansprüche 1 bis 6, **gekennzeichnet durch** ein Verhältnis der Höhe der Rippen (16) zum Außendurchmesser der Rohre (13) im Bereich von 1/10 bis 1/2.

8. Reaktor (1) nach einem der Ansprüche 1 bis 7, **gekennzeichnet durch** eine Dicke der Rippen (16) im Bereich zwischen 0,3 und 1,5 mm und **durch** eine Breite der Segmente (13) im Bereich zwischen 3 und 12 mm, bevorzugt in einem Bereich zwischen 4 und 8 mm.

9. Reaktor (1) nach einem der Ansprüche 1 bis 8, **dadurch gekennzeichnet, dass** das vorgeschaltete Lochblech (17) in einem Abstand zur Anströrnebene der ersten Reihe von Rippenrohren (19) durch den Einsatzgasstrom (2) entsprechend 7 bis 20 mal dem Durchmesser der Öffnungen (22) im vorgeschalteten Lochblech (17) angeordnet ist.

10. Reaktor (1) nach einem der Ansprüche 1 bis 9, **dadurch gekennzeichnet, dass** der Durchmesser der Öffnungen (22) im vorgeschalteten Lochblech (17) kleiner als die Hälfte des lichten Abstandes der Rippen (16) zwischen zwei aufeinander folgenden Umläufen ist.

11. Reaktor (1) nach einem der Ansprüche 1 bis 10, **dadurch gekennzeichnet, dass** das Öffnungsverhältnis im vorgeschalteten Lochblech (17) definiert als Summe der freien Flächen der Öffnungen (22) im vorgeschalteten Lochblech (17) bezogen auf die gesamte Querschnittsfläche senkrecht zur Zuführrichtung des Einsatzgasstromes (2) zur Einmischvorrichtung (12) ≤ 0,5, bevorzugt s 0.3, ist.

12. Reaktor (1) nach einem der Ansprüche 1 bis 11, **gekennzeichnet durch** ein Verhältnis der Lochblechdicke zum Durchmesser der Öffnungen (22, 23) im Lochblech (17, 18) im Bereich zwischen 0,75 und 2,0.

13. Reaktor (1) nach einem der Ansprüche 1 bis 12, **dadurch gekennzeichnet, dass** das nachgeschaltete Lochblech (18) von der Abströmebene der Rippenrohre (19) um 0,5 bis 2 mal dem Durchmesser der Rippenrohre (19) der letzten Reihe von Rippenrohren (19) beabstandet ist.

14. Reaktor (1) nach einem der Ansprüche 1 bis 13, **dadurch gekennzeichnet, dass** der Durchmesser der Öffnungen (23) im nachgeschalteten Lochblech (18) größer oder gleich dem Durchmesser der Öffnungen (22) im vorgeschalteten Lochblech (17) ist.

15. Reaktor (1) nach einem der Ansprüche 1 bis 14, **gekennzeichnet durch** einen Abstand des nachgeschalteten Lochblechs (18) zum Eintritt des Reaktionsgemisches in das Katalysatorwanderbett (4) entsprechend 5 bis 20 mal dem Durchmesser der Öffnungen (23) im nachgeschalteten Lochblech (18).

16. Verfahren zur Durchführung einer kontinuierlichen Oxidehydrierung eines Einsatzgasstromes (2) von gesättigten Kohlenwasserstoffen, **dadurch gekennzeichnet, dass** das Verfahren in einem Reaktor nach einem der Ansprüche 1 bis 15 durchgeführt wird.

17. Verfahren nach Anspruch 16, **dadurch gekennzeichnet, dass** der Einsatzgasstrom (2) ein Propan oder ein Butan enthaltender Gasstrom ist.

18. Verfahren nach Anspruch 16 oder 17, **dadurch gekennzeichnet, dass** der Sauerstoff enthaltende Gasstrom (3) Luft oder technisch reiner Sauerstoff ist.

19. Verfahren nach einem der Ansprüche 16 bis 18, **dadurch gekennzeichnet, dass** ein Teilstrom des Reaktionsgasgemisches aus dem in Strömungsrichtung letzten Reaktorabschnitt (9) in den ersten Reaktorabschnitt (9) recycliert wird.

20. Verfahren nach einem der Ansprüche 16 bis 19, **dadurch gekennzeichnet, dass** der Einsatzgasstrom, (2) mit dem den letzten Reaktorabschnitt (9) verlassenden Reaktionsgasgemisch durch indirekte Wärmeübertragung vorgewärmt wird.

## Claims

1. A reactor (1) for carrying out a continuous oxydehydrogenation of a feed gas stream (2) of saturated hydrocarbons after premixing with an oxygen-comprising gas stream (3) over a moving catalyst bed (4) which is introduced in the longitudinal direction of the reactor between two concentric cylindrical holding devices (5, 6) so as to leave a central interior space (7) and an intermediate space (8) between the moving catalyst bed (4) and the interior wall of the reactor to give a reaction gas mixture, wherein
the reactor (1) has two or more reactor sections (9) which are separated from one another by disk-shaped deflection plates (10) arranged alternately in the central interior space (7) and divided in subregions by annular deflection plates (11) arranged in the intermediate space between the moving catalyst bed (4) and the interior wall of the reactor, in each case with a mixing-in device (12) which is located upstream of the moving catalyst bed (4) in the flow direction of the reaction gas mixture and comprises the following elements:
- two or three rows arranged behind one another of tubes (13) which have turbulence generators on their outside which are finned tubes (19) having an external diameter of the tubes in the range from 25 to 50 mm, with the second row of finned tubes (19) being arranged so that the tubes are next to the gaps in the first row of finned tubes (19), and, in the case of three rows of finned tubes (19), with the third row of finned tubes (19) being arranged so that the tubes are next to the gaps in the second row of finned tubes (19), and which constrict the flow cross section for the feed gas stream (2) to from 1/2 to 1/10 of the free flow cross section, with the oxygen-comprising gas stream (3) being passed through the interior spaces of the tubes (13) and being injected via openings (14) in the tubes (13) into the feed gas stream (2), and with the openings (14) being arranged in the channels (15) between the fins (16) of the finned tubes (19), having in each case two openings (14) per channel (15) between the fins (16) of the finned tubes (19) in diametrically opposite positions on the channels (15), with the minimum distance to the adjacent finned tube (19) in the row of finned tubes, and having
- a perforated plate (17) upstream of the tubes (13) and a perforated plate (18) downstream of the tubes (13)
with the second, downstream perforated plate (18) having openings (14) whose diameter is greater than or equal to the diameter of the upstream perforated plate (17)
and the mixing-in device (5) having a depth, i.e. a distance between the upstream perforated plate (17) and the downstream perforated plate (18), in the range from 100 to 200 mm.

2. The reactor according to claim 1, wherein from 2 to 8 reactor sections (9), preferably four reactor sections (9), are provided.

3. The reactor (1) according to claim 1 or 2, wherein the finned tubes (19) constrict the free flow cross section for the feed gas stream (2) to from 1/3 to 1/6 of the original size.

4. The reactor (1) according to any of claims 1 to 3, wherein the finned tubes (19) are formed by tubes (19) which have a cylindrical exterior and have fins (16) which consist of elongated strips and are welded along a longitudinal edge of the strip in a spiral fashion onto the exterior of the tube and are cut with the exception of a fin base (21) to form segments (20).

5. The reactor (1) according to claim 4, wherein the segments (20) are rotated at an angle to the fin base (21).

6. The reactor (1) according to any of claims 1 to 5, wherein the finned tubes (19) have from 100 to 300 turns of the fins (9) per meter length of the finned tube (19).

7. The reactor (1) according to any of claims 1 to 6, wherein the ratio of the height of the fins (16) to the external diameter of the tubes (13) is in the range from 1/10 to 1/2.

8. The reactor (1) according to any of claims 1 to 7 wherein the thickness of the fins (16) is in the range from 0.3 to 1.5 mm and the width of the segments (13) is in the range from 3 to 12 mm, preferably in the range from 4 to 8 mm.

9. The reactor (1) according to any of claims 1 to 8, wherein the upstream perforated plate (17) is arranged at a distance corresponding to from 7 to 20 times the diameter of the openings (22) in the upstream perforated plate (17) from the inflow plane of the first row of finned tubes (19) for the feed gas stream (2).

10. The reactor (1) according to any of claims 1 to 9, wherein the diameter of the openings (22) in the upstream perforated plate (17) is smaller than half of the clear spacing of the fins (16) between two successive turns.

11. The reactor (1) according to any of claims 1 to 10, wherein the opening ratio in the upstream perforated plate (17) defined as the sum of the free areas of the openings (22) in the upstream perforated plate (17) based on the total crosssectional area perpendicular to the inflow direction of the feed gas stream (2) into the mixing-in device (12) is ≤ 0.5, preferably ≤ 0.3.

12. The reactor (1) according to any of claims 1 to 11 in which the ratio of the perforated plate thickness to the diameter of the openings (22, 23) in the perforated plate (17, 18) is in the range from 0.75 to 2.0.

13. The reactor (1) according to any of claims 1 to 12, wherein the downstream perforated plate (18) is located at a distance of from 0.5 to 2 times the diameter of the finned tubes (19) of the last row of finned tubes (19) from the outflow plane of the finned tubes (19).

14. The reactor (1) according to any of claims 1 to 13, wherein the diameter of the openings (23) in the downstream perforated plate (18) is greater than or equal to the diameter of the openings (22) in the upstream perforated plate (17).

15. The reactor (1) according to any of claims 1 to 14 in which the distance from the downstream perforated plate (18) to the entry of the reaction mixture into the moving catalyst bed (4) corresponds to from 5 to 20 times the diameter of the openings (23) in the downstream perforated plate (18).

16. A process for carrying out a continuous oxydehydrogenation of a feed gas stream (2) of saturated hydrocarbons, wherein the process is carried out in a reactor according to any of claims 1 to 15.

17. The process according to claim 16, wherein the feed gas stream (2) is a gas stream comprising propane or butane.

18. The process according to claim 16 or 17, wherein the oxygen-comprising gas stream (3) is air or technical grade oxygen.

19. The process according to any of claims 16 to 18, wherein a substream of the reaction gas mixture from the last reactor section (9) in the flow direction is recycled into the first reactor section (9).

20. The process according to any of claims 16 to 19, wherein the feed gas stream (2) is preheated by indirect heat transfer from the reaction gas mixture leaving the last reactor section (9).

## Revendications

1. Réacteur (1) pour la réalisation d'une oxydéshydrogénation continue d'un courant gazeux d'entrée (2) d'hydrocarbures saturés après un mélange préliminaire avec un courant gazeux contenant de l'oxygène (3) sur un lit catalytique mobile (4), qui est disposé dans la direction longitudinale du réacteur entre deux dispositifs d'ancrage cylindriques concentriques (5, 6) en laissant une chambre intérieure centrale (7) et une chambre intermédiaire (8) entre le lit catalytique mobile (4) et l'enveloppe intérieure du réacteur, pour obtenir un mélange réactionnel gazeux, **caractérisé en ce que**
le réacteur (1) comprend deux sections de réacteur ou plus (9), qui sont séparées les unes des autres en zones partielles avec en alternance des chicanes en forme de plaques (10) agencées dans la chambre intérieure centrale (7) et des chicanes annulaires (11) agencées dans la chambre intermédiaire entre le lit catalytique mobile (4) et l'enveloppe intérieure du réacteur, avec à chaque fois un dispositif de mélange (12) disposé avant le lit catalytique mobile (4) dans la direction de l'écoulement du mélange réactionnel gazeux, qui est formé par les éléments suivantes :
- deux ou trois séries successives de tubes (13) comprenant des générateurs de turbulence sur leur côté extérieur, qui sont des tubes à ailettes (19) ayant un diamètre extérieur des tubes dans la plage comprise entre 25 et 50 mm, la deuxième série de tubes à ailettes (19) étant agencée au niveau des espaces de la première série de tubes à ailettes (19) et, dans le cas de trois séries de tubes à ailettes (19), la troisième série de tubes à ailettes (19) étant agencée au niveau des espaces de la deuxième série de tubes à ailettes (19), et qui rétrécissent la section de passage pour le courant gazeux d'entrée (2) à 1/2 à 1/10 de la section de passage libre, le courant gazeux contenant de l'oxygène (3) étant conduit dans les espaces intérieurs des tubes (13) et étant injecté dans le courant gazeux d'entrée (2) par des ouvertures (14) des tubes (13), et les ouvertures (14) étant agencées dans les spires d'ailettes (15) entre les ailettes (16) des tubes à ailettes (19), avec à chaque fois deux ouvertures (14) par spire d'ailettes (15) entre les ailettes (16) des tubes à ailettes (19), à des positions diamétralement opposée des spires d'ailettes (15), à la distance la plus courte au tube à ailettes respectivement voisin (19) dans la série de tubes à ailettes, ainsi que
- une plaque perforée (17) en amont des tubes (13) et une plaque perforée (18) en aval des tubes (13),
la seconde plaque perforée en aval (18) comprenant des ouvertures (14), dont le diamètre est supérieur ou égal au diamètre de la plaque perforée en amont (17),
et le dispositif de mélange (5) présentant une profondeur, c.-à-d. une distance entre la plaque perforée en amont (17) et la plaque perforée en aval (18) dans la plage comprise entre 100 et 200 mm.

2. Réacteur selon la revendication 1, **caractérisé en ce que** deux à huit sections de réacteur (9), de préférence quatre sections de réacteur (9), sont prévues.

3. Réacteur (1) selon la revendication 1 ou 2, **caractérisé en ce que** les tubes à ailettes (19) rétrécissent la section de passage libre pour le courant gazeux d'entrée (2) à 1/3 à 1/6 de celle-ci.

4. Réacteur (1) selon l'une quelconque des revendications 1 à 3, **caractérisé en ce que** les tubes à ailettes (19) sont formés par des tubes (19) à circonférence extérieure cylindrique, comprenant des ailettes (16) constituées de bandes longitudinales, soudées en spirale sur celle-ci le long d'un bord longitudinal en bande, qui sont découpées à l'exception d'une base d'ailette (21) pour former des segments (20).

5. Réacteur (1) selon la revendication 4, **caractérisé en ce que** les segments (20) sont tournés à un angle par rapport à la base d'ailette (21).

6. Réacteur (1) selon l'une quelconque des revendications 1 à 5, **caractérisé en ce que** les tubes à ailettes (19) comprennent 100 à 300 révolutions des ailettes (9) par mètre de longueur de tube à ailettes (19).

7. Réacteur (1) selon l'une quelconque des revendications 1 à 6, **caractérisé par** un rapport entre la hauteur des ailettes (16) et le diamètre extérieur des tubes (13) dans la plage allant de 1/10 à 1/2.

8. Réacteur (1) selon l'une quelconque des revendications 1 à 7, **caractérisé par** une épaisseur des ailettes (16) dans la plage comprise entre 0,3 et 1,5 mm et par une largeur des segments (13) dans la plage comprise entre 3 et 12 mm, de préférence dans une plage comprise entre 4 et 8 mm.

9. Réacteur (1) selon l'une quelconque des revendications 1 à 8, **caractérisé en ce que** la plaque perforée en amont (17) est agencée à une distance du plan d'incidence de la première série de tubes à ailettes (19) par le courant gazeux d'entrée (2) correspondant à 7 à 20 fois le diamètre des ouvertures (22) dans la plaque perforée en amont (17).

10. Réacteur (1) selon l'une quelconque des revendications 1 à 9, **caractérisé en ce que** le diamètre des ouvertures (22) dans la plaque perforée en amont (17) est inférieur à la moitié de la distance intérieure des ailettes (16) entre deux révolutions successives.

11. Réacteur (1) selon l'une quelconque des revendications 1 à 10, **caractérisé en ce que** le rapport d'ouverture dans la plaque perforée en amont (17) défini comme la somme des surfaces libres des ouvertures (22) dans la plaque perforée en amont (17) sur la surface de section totale perpendiculaire à la direction d'introduction du courant gazeux d'entrée (2) dans le dispositif de mélange (12) est ≤ 0,5, de préférence ≤ 0,3.

12. Réacteur (1) selon l'une quelconque des revendications 1 à 11, **caractérisé par** un rapport entre l'épaisseur de la plaque perforée et le diamètre des ouvertures (22, 23) dans la plaque perforée (17, 18) dans la plage comprise entre 0,75 et 2,0.

13. Réacteur (1) selon l'une quelconque des revendications 1 à 12, **caractérisé en ce que** la plaque perforée en aval (18) est espacée du plan d'échappement des tubes à ailettes (19) de 0,5 à 2 fois le diamètre des tubes à ailettes (19) de la dernière série de tubes à ailettes (19).

14. Réacteur (1) selon l'une quelconque des revendications 1 à 13, **caractérisé en ce que** le diamètre des ouvertures (23) dans la plaque perforée en aval (18) est supérieur ou égal au diamètre des ouvertures (22) dans la plaque perforée en amont (17).

15. Réacteur (1) selon l'une quelconque des revendications 1 à 14, **caractérisé par** une distance entre la plaque perforée en aval (18) et l'entrée du mélange réactionnel dans le lit catalytique mobile (4) correspondant à 5 à 20 fois le diamètre des ouvertures (23) dans la plaque perforée en aval (18).

16. Procédé de réalisation d'une oxydéshydrogénation continue d'un courant gazeux d'entrée (2) d'hydrocarbures saturés, **caractérisé en ce que** le procédé est réalisé dans un réacteur selon l'une quelconque des revendications 1 à 15.

17. Procédé selon la revendication 16, **caractérisé en ce que** le courant gazeux d'entrée (2) est un courant gazeux contenant du propane ou du butane.

18. Procédé selon la revendication 16 ou 17, **caractérisé en ce que** le courant gazeux contenant de l'oxygène (3) est de l'air ou de l'oxygène techniquement pur.

19. Procédé selon l'une quelconque des revendications 16 à 18, **caractérisé en ce qu'**une partie du mélange réactionnel gazeux issu de la dernière section de réacteur (9) dans la direction de l'écoulement est recyclée dans la première section de réacteur (9).

20. Procédé selon l'une quelconque des revendications 16 à 19, **caractérisé en ce que** le courant gazeux d'entrée (2) est préchauffé avec le mélange réactionnel gazeux quittant la dernière section de réacteur (9) par transfert de chaleur indirect.
